(19) Europäisches Patentamt
European Patent Office
Office européen des brevets

(11) Publication number: **0 112 633**
**A2**

# (12) EUROPEAN PATENT APPLICATION

(21) Application number: **83306792.9**

(22) Date of filing: **08.11.83**

(51) Int. Cl.[3]: **C 07 C 177/00**, C 07 F 7/18, A 61 K 31/557

(30) Priority: **20.12.82 JP 221995/82**
**28.12.82 JP 227330/82**
**22.02.83 JP 26899/83**

(43) Date of publication of application: **04.07.84**
**Bulletin 84/27**

(84) Designated Contracting States: **CH DE FR GB IT LI SE**

(71) Applicant: **TEIJIN LIMITED, 11, 1-Chome, Minamihonmachi Higashi-ku, Osaka-shi Osaka (JP)**

(72) Inventor: **Hazato, Atsuo, Teijin Musashino-ryo 3-5-18, Tamadaira, Hino-shi Tokyo 191 (JP)**
Inventor: **Tanaka, Toshio, Teijin Toyota Apr. No. 10 5-15-6, Tamadaira, Hino-shi 191 Tokyo (JP)**
Inventor: **Kurozumi, Seizi, 1-2-28, Higashitokura, Kokubunji-shi Tokyo 185 (JP)**

(74) Representative: **Votier, Sidney David et al, CARPMAELS & RANSFORD 43, Bloomsbury Square, London WC1A 2RA (GB)**

## (54) Novel 5-thiaprostaglandin, process for production thereof, and pharmaceutical use thereof.

(57) The present invention relates to novel 5-thiaprostaglandin which include 5-thiaprostaglandin $E_1$ derivative, $\Delta^7$-5-thiaprostaglandin $E_1$, 5-thiaprostaglandin $A_1$, and $\Delta^7$-5-thiaprostaglandin $A_1$; to process for producing these compounds; and to pharmaceutical uses of these compounds, especially as anti-ulcer or antitumor agent.

EP 0 112 633 A2

# NOVEL 5-THIAPROSTAGLANDIN, PROCESS FOR PRODUCTION THEREOF, AND PHARMACEUTICAL USE THEREOF

## BACKGROUND OF THE INVENTION

### Field of the Invention

The present invention relates to novel 5-thiaprostaglandin, processes for their production, and their pharmaceutical use. More specifically, The present invention relates to novel 5-thiaprostaglandin which include 5-thiaprostaglandin $E_1$ derivative, $\Delta^7$-5-thiaprostaglandins $E_1$, 5-thiaprostaglandins $A_1$ and $\Delta^7$-5-thiaprostaglandins $A_1$; to processes for producing these compounds; and to pharmaceutical uses of these compounds, especially as anti-ulcer or antitumor agent.

### Description of the Prior Art

Natural prostaglandins are known as local hormones having high biological and pharmacological activities, accordingly many works have been done on derivatives of natural prostaglandins. In particular, prostaglandin $E_1$ have potent platelet aggregation inhibiting activity and strong vasodilating activity, and are expected to find clinical applications.

Various investigations have been undertaken previously on artificial prostaglandins resulting from substitution of a sulfur atom for one or two carbon atoms forming the skeleton of natural prostaglandins. Examples of such known artificial prostaglandins, are shown below.

(1) 1S-Prostaglandins $E_2$ or $F_{2\alpha}$ [J. Org. Chem., 40, 521 (1975) and Japanese Laid-Open Patent Publication No. 34747 / 1978] having a skeleton resulting

from substitution of a sulfur atom for the carbon atom at the 1-position.

(2) 3S-11-Deoxyprostaglandins $E_1$ [Tetrahedron Letters, 1975, 765 and J. Med. Chem., 20, 1662 (1977)].

(3) 7S-Prostaglandins $F_{1\alpha}$ [J. Amer. Chem. Soc., 96, 6757, (1974)].

(4) 9S-Prostaglandins $E_1$ [Tetrahedron Letters, 1974, 4267 and 4459, ibid. 1976, 4793, and Heterocycles, 6, 1097 (1977)].

(5) 11S-Prostaglandins $E_1$ or $F_{1\alpha}$ [Tetrahedron Letters, 1975, 1165].

(6) 13S-Prostaglandins E or F (U.S. Patent No. 4,080,458).

(7) 15S-Prostaglandin $E_2$ (Tetrahedron Letters, 1977, 1629).

(8) 11-Deoxy-7S-prostaglandins $E_1$ and 7S, 13S-prostaglandins (U.S. Patent No. 4,180,672).

(9) 7S-, 6S-, or 4S-Prostaglandins (European Laid-Open Patent Publication No. 0051284$A_1$).

Abstracts of 103th meeting of Pharmaceutical Society of Japan (published on March, 10, 1983) discloses a 5-thiaprostaglandin $E_1$ methyl ester, $\Delta^7$-5-thiaprostaglandin $E_1$ methyl ester 11, 15-bis (t-butyldimethylsilyl) ether, and processes for producing these compounds. This literature, however, dosen't disclose pharmacological activities of these compounds.

## SUMMARY OF THE INVENTION

It is an object of this invention to provide novel 5-thiaprostaglandin.

Another object of this invention to provide novel 5-thiaprostaglandin which is useful in inhibiting ulcer formation, or in treatment of malignant tumors.

Still another object of this invention is to provide novel 5-thiaprostaglandin which has better selective biological activities than natural $PGE_1$.

Yet another object of this invention is to provide processes for producing the novel 5-thiaprostaglandin of the invention.

Other objects and advantages of the invention will become apparent from the following description.

These objects and advantages are achieved in accordance with this invention by a 5-thiaprostaglandin represented by the following formula

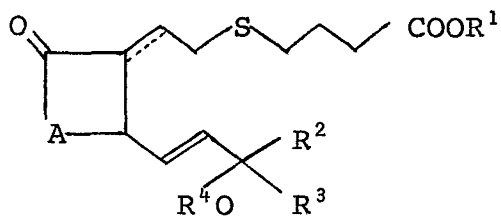

(1)

wherein A represents $-CH_2-CH(OR^5)-$ or $-CH=CH-$ (cis) in which $R^5$ represents a hydrogen atom, a tri($C_1$-$C_7$)hydrocarbon-silyl group, or a group forming an acetal linkage together with the oxygen atom of the hydroxyl group, the symbol ┄┄ represents, a single or double bond, $R^1$ represents a hydrogen atom, a $C_1$-$C_{10}$ linear or branched alkyl group, or one equivalent of a cation, $R^2$ represents a hydrogen atom, or methyl group, $R^3$ represents an unsubstituted $C_3$- $C_8$ linear or branched alkyl group, a substituted $C_1$- $C_5$ linear or branched alkyl group substituted by a substituent selected from phenyl, phenoxy, $C_1$-$C_6$ alkoxy, $C_5$-$C_6$ cycloalkyl, which substituent may be

substituted, or a substituted or unsubstituted alicyclic group, $R^4$ represents a hydrogen atom, a tri$(C_1-C_7)$hydrocarbon-silyl group, or a group forming an acetal linkage together with the oxygen atom of the hydroxyl group; provided that when A is $-CH_2-\underset{OR^5}{CH}-$ and $R^2$ is a hydrogen atom, $R^3$ is not an unsubstituted $C_3-C_8$ linear alkyl group.

DESCRIPTION ON THE PREFERED EMBODIMENTS

The 5-thiaprostaglandin of the present invention is expressed by the aforementioned formula (1).

In the formula (1) A represents a $-CH_2-\underset{OR^5}{CH}-$ or $-CH=CH-$(cis) in which $R^5$ represents a hydrogen atom, a tri$(C_1-C_7)$hydrocarbon-silyl group, or a group forming an acetal linkage together with the oxygen atom of the hydroxyl group. For the sake of convenience, the 5-thiaprostaglandin of this invention can be divided into the following two groups according to the definition of A.

(a) 5-thiaprostaglandin of formula (1)-a

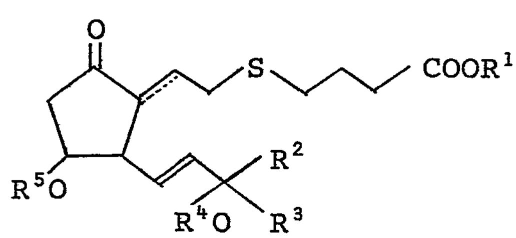

(1)-a

wherein $R^1$, $R^2$, $R^3$, $R^4$, $R^5$ and symbol ===== are defined as above.

(b) 5-thiaprostaglandin of formula (1)-b

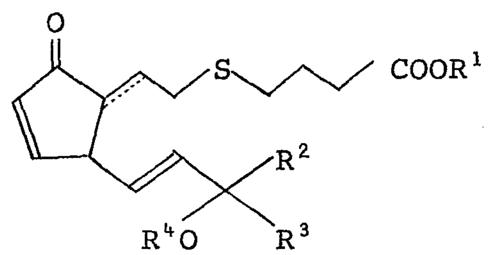

(1)-b

wherein $R^1$, $R^2$, $R^3$, $R^4$ and symbol ┅┅┅ are defined as above.

In the formula (1) the symbol ┅┅┅ represents a single or double bond.

In the formula (1) $R^1$ represents a hydrogen atom, a $C_1$-$C_{10}$ linear or branched alkyl group, or one equivalent of a cation.

Examples of the $C_1$-$C_{10}$ linear or branched alkyl group include methyl, ethyl, n-propyl, iso-propyl, n-butyl, sec-butyl, tert-butyl, n-pentyl, n-hexyl, n-heptyl, n-octyl, n-nonyl and n-decyl.

Examples of one equivalent of a cation include ammonium cations such as $NH_4^+$, or a tetramethyl ammonium, monomethyl ammonium, dimethyl ammonium, trimethyl ammonium, benzyl ammonium, phenethyl ammonium, morpholinium, monoethanol ammonium, or piperidinium cation; alkali metal cations such as $Na^+$ or $K^+$; and divalent or trivalent metal cations such as $1/2Ca^{2+}$, $1/2Mg^{2+}$, $1/2Zn^{2+}$, and $1/3Al^{3+}$.

In the formula (1) $R^3$ represents an unsubstituted $C_3$-$C_8$ linear or branched alkyl group; a substituted $C_1$-$C_5$ linear or branched alkyl group substituted by a substituent selected from a phenyl group, a phenoxy group, a $C_1$-$C_6$ alkoxy group and a $C_5$-$C_6$ cycloalkyl group, which substituent may be substituted; or a substituted or unsubstituted alicyclic group; provided that when A is

$-CH_2-\underset{OR^5}{\underset{|}{C}}H-$ and $R^2$ is a hydrogen atom, $R^3$ is not an unsubstituted $C_3$-$C_8$ linear alkyl group. The unsubstituted $C_3$-$C_8$ linear or branched alkyl group includes, for example, propyl, n-butyl, n-pentyl, n-hexyl, 2-methyl-1-hexyl, 2-methyl-2-hexyl, n-heptyl, and n-octyl. The n-pentyl, n-hexyl, 2-methyl-1-hexyl, and 2-methyl-2-hexyl are preferred. The substituted $C_1$-$C_5$ linear or branched alkyl group includes, for example, methyl, ethyl, n-propyl, iso-propyl, n-butyl, sec-butyl, t-butyl and n-pentyl. These alkyl groups are substituted by a phenyl group; a phenoxy group; a $C_1$-$C_6$ alkoxy group such as methoxy, ethoxy, n-propoxy, iso-propoxy, n-butoxy, iso-butoxy, t-butoxy, n-pentoxy, or n-hexoxy; or a $C_5$-$C_6$ cycloalkyl group such as cyclopentyl or cyclohexyl.

These substituents may be substituted by the substituents. Examples of suitable substituents are halogen atoms, a hydroxyl group, $C_2$-$C_7$ acyloxy groups, $C_1$-$C_4$ alkoxy groups which may be halogenated, a nitrile group, a carboxyl group and ($C_1$-$C_6$) alkoxycarbonyl groups. The halogen atoms are fluorine, chlorine and bromine atoms, and fluorine and chlorine atoms are preferred. Examples of the $C_2$-$C_7$ acyloxy groups are acetoxy, propionyloxy, n-butyryloxy, iso-butyryloxy, n-valeryloxy, iso-valeryloxy, caproyloxy, enanthyloxy or benzoyloxy.

Examples of suitable $C_1$-$C_4$ linear or branched alkyl groups which may be halogenated include methyl, ethyl, n-propyl, iso-propyl, n-butyl, chloromethyl, dichloromethyl, and tri-fluoromethyl.

Examples fo suitable $C_1$-$C_4$ alkoxy groups which may be halogenated include methoxy, ethoxy, n-propoxy, iso-propoxy, n-butoxy, chloromethoxy, dichloromethoxy, tri-fluoromethoxy.

Examples of the ($C_1$-$C_6$)alkoxycarbonyl group

are methoxycarbonyl, ethoxycarbonyl, butoxycarbonyl and hexyloxycarbonyl.

The substituted or unsubstituted alicyclic group may be a saturated or unsaturated $C_5$-$C_8$, preferably $C_5$-$C_6$, especially preferably $C_6$ group, such as cyclopentyl, cyclohexyl, cyclohexenyl, cycloheptyl, or cyclooctyl, which is unsubstituted or substituted by the same substituents as exemplified thereabove.

Preferably, $R^3$ is n-pentyl, n-hexyl, 2-methyl-1-hexyl, 2-methyl-2-hexyl, cyclopentyl or cyclohexyl.

In the formula (1) and (1)-a, $R^4$ and $R^5$ are identical or different, and each represents a hydrogen atom, a tri($C_1$-$C_7$)hydrocarbonsilyl group, or a group forming an acetal linkage together with the oxygen atom of the hydroxyl group. Examples of suitable tri($C_1$-$C_7$)hydrocarbon-silyl groups include tri($C_1$-$C_4$)alkylsilyl groups such as trimethylsilyl, triethylsilyl or t-butyldimethylsilyl, ($C_1$-$C_4$)alkyldiphenylsilyl groups such as t-butyldiphenylsilyl, and a tribenzylsilyl group. Examples of the group forming an acetal linkage with the oxygen atom of the hydroxyl group are methoxymethyl, 1-ethoxyethyl, 2-methoxy-2-propyl, 2-ethoxy-2-propyl, (2-methoxyethoxy)methyl, benzyloxymethyl, 2-tetrahydropyranyl, 2-tetrahydrofuranyl and 6,6-dimethyl-3-oxa-2-oxo-bicyclo[3.1.0]hex-4-yl. Of these, 2-tetrahydropyranyl, 2-tetrahydrofuranyl, 1-ethoxyethyl, 2-methoxy-2-propyl, and (2-methoxyethoxy)methyl, and 6,6-dimethyl-3-oxa-2-oxo-bicyclo[3.1.0]hex-4-yl are preferred.

It should be understood that the hydrocarbon-silyl group and the group forming an acetal linkage are protective groups for the hydroxyl group. The hydrocarbon-silyl group or the acetal-forming group can be easily removed under acidic to neutral conditions.

Preferred as $R^4$ or $R^5$ are a hydrogen atom, a tri($C_1$-$C_4$)alkylsilyl group, a ($C_1$-$C_4$)alkyldiphenyl silyl group, a 2-tetrahydropyranyl group, a 2-tetrahydrofuranyl group, a 1-ethoxyethyl group, a 2-ethoxy-3-propyl group, a (2-methoxyethoxy)methyl group, and a 6,6-dimethyl-3-oxa-2-oxo-bycyclo[3.1.0] hex-4-yl group.

The 5-thiaprostaglandin expressed by the formula (1) of the present invention contain asymmetric carbon atoms in its molecule and the present invention involves all the stereoisomers or optical isomers, which arise from these asymmetric carbon atoms, and mixtures thereof.

Of all these isomers, natural-type 5-thiaprostaglandin expressed by the following formula (1)-a'

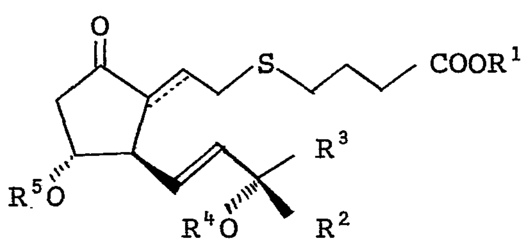

(1)-a'

wherein $R^1$, $R^2$, $R^3$, $R^4$, $R^5$ and symbol ┅┅ are defined as above,

or natural-type 5-thiaprostaglandin expressed by the following formula (1)-b'

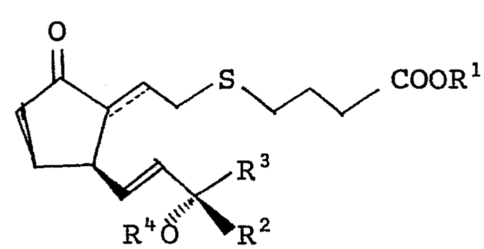

(1)-b'

wherein $R^1$, $R^2$, $R^3$, $R^4$ and symbol ┈┈ are defined as above,

are preferable.

(a) Specific examples of the 5-thiaprostaglandin of formula (1)-a are given below.

(100) 20-methyl-5-thiaprostaglandin $E_1$
(102) 20-ethyl-5-thiaprostaglandin $E_1$
(104) 16-methyl-5-thiaprostaglandin $E_1$
(106) 15-methyl-5-thiaprostaglandin $E_1$
(108) 17-methyl-5-thiaprostaglandin $E_1$
(110) 17,20-dimethyl-5-thiaprostaglandin $E_1$
(112) 20-ethyl-17-methyl-5-thiaprostaglandin $E_1$
(114) 17(S),20-dimethyl-5-thiaprostaglandin $E_1$
(116) 17(R),20-dimethyl-5-thiaprostaglandin $E_1$
(118) 16,17,18,19,20-pentanor-15-cyclohexyl-5-thiaprostaglandin $E_1$
(120) 16,17,18,19,20-pentanor-15-cyclopentyl-5-thiaprostaglandin $E_1$
(122) 17,18,19,20-tetranor-16-phenoxy-5-thiaprostaglandin $E_1$
(124) 17,20-dimethyl-5-thiaprostaglandin $E_1$
(126) 16(S),20-dimethyl-5-thiaprostaglandin $E_1$
(128) 16(R),20-dimethyl-5-thiaprostaglandin $E_1$
(130) 16,17,18,19,20-pentanor-15-cyclohexenyl-5-thiaprostaglandin $E_1$
(132) 16,17,18,19,20-pentanor-15-cyclooctyl-5-thiaprostaglandin $E_1$
(134) 17,20-dimethyl-5-thia-$\Delta^7$-prostaglandin $E_1$
(136) 15-methyl-5-thia-$\Delta^7$-prostaglandin $E_1$
(138) 17(S),20-dimethyl-5-thia-$\Delta^7$-prostaglandin $E_1$
(140) 17(R),20-dimethyl-5-thia-$\Delta^7$-prostaglandin $E_1$
(142) 20-methyl-5-thia-$\Delta^7$-prostaglandin $E_1$
(144) 20-ethyl-5-thia-$\Delta^7$-prostaglandin $E_1$

(146) 16,17,18,19,20-pentanor-15-cyclohexyl-5-thia-$\Delta^7$-prostaglandin $E_1$

(148) 16,17,18,19,20-pentanor-15-cyclopentyl-5-thia-$\Delta^7$-prostaglandin $E_1$

(150) 16,17,18,19,20-pentanor-15-cyclohexyl-5-thia-$\Delta^7$-prostaglandin $E_1$

(152) methyl ester of (100)

(154) methyl ester of (102)

(156) methyl ester of (104)

(158) ethyl ester of (110)

(160) n-propyl ester of (112)

(162) n-propyl ester of (120)

(164) sodium salt of (122)

(166) sodium salt of (124)

(168) potassium salt of (130)

(170) ammonium salt of (134)

(172) morpholium salt of (140)

(174) ethanol ammonium salt of (142)

(176) 11,15-bis(t-butyldimethylsilyl) ether of (144)

(178) 11,15-bis(2-tetrahydropyraryl) ether of (146)

(180) 11,15-bis(2-tetrahydrofuranyl) ether of (148)

(b) Specific example of the 5-thiaprostaglandin of formula (1)-b are given below.

(200) 5-thiaprostaglandin $A_1$

(202) 20-methyl-5-thiaprostaglandin $A_1$

(204) 16-methyl-5-thiaprostaglandin $A_1$

(206) 15-methyl-5-thiaprostaglandin $A_1$

(208) 17-methyl-5-thiaprostaglandin $A_1$

(210) 17(S),20-dimethyl-5-thiaprostaglandin $A_1$

(212) 17(R),20-dimethyl-5-thiaprostaglandin $A_1$

(214) 20-ethyl-5-thiaprostaglandin $A_1$

(216) 16,17,18,19,20-pentanor-15-cyclohexyl-5-thia-prostaglandin $A_1$

(218) 16,17,18,19,20-pentanor-15-cyclopentyl-5-thia-protaglandin $A_1$

(220) 17,18,19,20-tetranor-16-phenoxy-5-thia-prostaglandin $A_1$

(222) 16,17,18,19,20-pentanor-15-cyclooctyl-5-thiaprostaglandin $A_1$

(224) 16,17,18,19,20-pentanor-15-cyclohexenyl-5-thiaprostaglandin $A_1$

(226) 5-thia-$\Delta^7$-prostaglandin $A_1$

(228) 15-methyl-5-thia-$\Delta^7$-prostaglandin $A_1$

(230) 16-methyl-5-thia-$\Delta^7$-prostaglandin $A_1$

(232) 17(S),20-dimethyl-5-thia-$\Delta^7$-prostaglandin $A_1$

(234) 17(R),20-dimethyl-5-thia-$\Delta^7$-prostaglandin $A_1$

(236) 20-ethyl-5-thia-$\Delta^7$-prostaglandin $A_1$

(238) 16,17,18,19,20-pentanor-15-cyclopentyl-5-thia-$\Delta^7$-prostaglandin $A_1$

(240) 16,17,18,19,20-pentanor-15-cyclohexyl-5-thia-$\Delta^7$-prostaglandin $A_1$

(242) methyl ester of (200)

(244) methyl ester of (202)

(246) ethyl ester of (204)

(248) n-propyl ester of (206)

(250) n-butyl ester of (208)

(252) sodium salt of (210)

(254) sodium salt of (214)

(256) sodium salt of (218)

(258) potassium salt of (220)

(260) ammonium salt of (224)

(262) 11,15-bis(t-butyldimethylsilyl) ether of (230)

(264) 11,15-bis(2-tetrahydropyranyl) ether of (234)

The 5-thiaprostaglandin (when the symbol ------ between the 7- and 8-positions indicates a double bond) of the following formula (1)-a-1

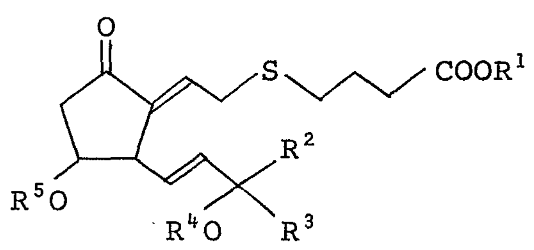

(1)-a-1

wherein $R^1$, $R^2$, $R^3$, $R^4$ and $R^5$ are defined as above,

are produced in accordance with the present invention by reacting a 7-hydroxy-5-thiaprostaglandin of the following formula (11)

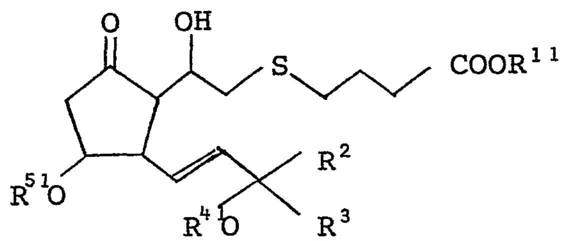

(11)

where $R^{11}$ represents a $C_1$-$C_{10}$ linear or branched alkyl group, $R^{41}$ and $R^{51}$ represents a tri($C_1$-$C_7$)hydrocarbonsilyl group or a group forming an acetal linkage together with the oxygen atom of the hydroxyl group, $R^2$ and $R^3$ are defined as above,

with a reactive derivative of an organic sulfonic acid in the presence of a basic compound to form the corresponding 7-organic sulfonyloxyprostaglandin, treating the 7-organic sulfoxyprostaglandin in the presence of a basic compound, and if required, subjecting the reaction product to a reaction of removing the protective group, a reaction of hydrolysis, or a salt-forming reaction.

The 7-hydroxy-5-thiaprostaglandin of formula (11) is compound which do not contain active hydrogen atom in the $R^{11}$, $R^{41}$ and $R^{51}$, and do not contain one equivalent of a cation in the $R^{11}$, as is seen from the definitions of $R^{11}$, $R^{41}$, $R^{51}$ in formula (11)

The 7-hydroxy-5-thiaprostaglandin of the formula (11) can be produced by a process comprising reacting the corresponding cyclopent-2-en-1-ones with the corresponding organolithium compounds in the presences

of cuprous salt and reacting the resulting β-substituted enolates with the corresponding aldehydes in accordance with the same way as described in the specification of U.S. Patent No. 4,315,032. U.S. Patent No. 4,315,032 is hereby cited as reference.

According to the process of this invention, the 7-hydroxy-5-thiaprostaglandin of formula (ll) is first reacted with a reactive derivative of an organic sulfonic acid in the presence of a basic compound to give the corresponding 7-organic sulfonyloxyprostaglandin.

Proffered reactive derivative of an organic sulfonic acid are organic sulfonic acid halides and organic sulfonic acid anhydrides.

Examples of the organic sulfonic acid halides include methanesulfonyl chloride, ethanesulfonyl bromide, n-butanesulfonyl chloride, tert-butanesulfonyl chloride, trifluoromethanesulfonyl chloride, nonafluorobutanesulfonyl chloride, benzenesulfonyl chloride, p-toluenesulfonyl chloride, 4-bromobenzenesulfonyl chloride, pentafluorobenzenesulfonyl chloride, 4-chloro-3-nitrobenzenesulfonyl chloride, 2,3,4-trichlorobenzenesulfonyl chloride, and 2-phenylethanesulfonyl chloride. Of these, methanesulfonyl chloride, trifluoromethanesulfonyl chloride, benzenesulfonyl chloride, and p-toluenesulfonyl chloride are preferred.

Specific examples of the organic sulfonic acid anhydride are methanesulfonic anhydride, ethanesulfonic anhydride, trifluoromethanesulfonic anhydride, nonafluorobutanesulfonic anhydride, benzenesulfonic anhydride, p-toluenesulfonic anhydride, trifluoromethanesulfonic-benzenesulfonic mixed anhydride, trifluoromethanesulfonic-p-toluenesulfonic mixed anhydride, and trifluoromethanesulfonic-4-nitrobenzenesulfonic mixed anhydride. Of these, methanesulfonic anhydride,

trifluoromethanesulfonic anhydride and p-toluenesulfonic anhydride are preferred.

As the basic compound, organic amines, especially tertiary organic amines, are preferably used.

Examples of the organic amines include trimethylamine, triethylamine, isopropyldimethyamine, diisopropylcyclohexylamine, diisopropylethylamine, pyridine, 2,6-lutidine, 2,4,6-collidine, 4-(N,N-dimethyl)aminopyridine, 1,5-diazabicyclo [4.3.0]non-5-ene, and 1,4-diazabicyclo[2.2.2]octane. Of these, triethylamine, pyridine, 2,6-lutidine, and 4-(N,N-dimethyl)aminopyridine are preferred.

The reaction is carried out usually in an aprotic inert organic solvent. Examples of the solvent include halogenated hydrocarbons such as dichloromethane, chloroform, carbon tetrachloride and 1,2-dichloroethane; ethers such as ethyl ether, tetrahydrofuran and dioxane; aromatic hydrocarbons such as benzene, toluene and xylene; aliphatic hydrocarbons such as hexane, pentane and cyclohexane; esters such as ethyl acetate; dimethylformamide; dimethyl sulfoxide; and hexamethyl phosphoramide. Of these, the halogenated hydrocarbons are preferred.

The above reaction is between the hydroxyl group at the 7-position of the 7-hydroxy-5-thiaprostaglandin of formula (11) and the reactive derivative of the organic sulfonic acid, and therefore, stoichiometrically, the two compounds react in equimolar proportions. The reaction proceeds, however, even when one of the starting compounds is used in an amount stoichiometrically less than the other. Hence, the use of these materials in such proportions may be permissible. In practice, about 1 to about 10 moles of the reactive derivative of the organic sulfonic acid is used per mole of the 7-hydroxy-5-thiaprostaglandin of general formula (11). Generally, when the rate of the

reaction is slow, the reactive derivative of the organic sulfonic acid is used in excess.

The basic compound is used in an amount of at least about 1 mole, preferably at least 2 moles, per mole of the reactive derivative of the organic sulfonic acid. The basic compound may be used in excess, for example in such a large excess as to serve as a reaction solvent.

The reaction is carried out usually at 0 to 100°C, preferably 0 to 50°C, especially preferably 10 to 30°C. The reaction comes to an end usually in 0.5 to 10 hours. The progress of the reaction can be confirmed by monitering the disappearance of the starting compounds by, for example, thin-layer chromatography.

Thus, according to the aforesaid first reaction, there is formed a 7-organic sulfonyloxy prostaglandin resulting from the conversion of the hydroxyl group at the 7-position of the 7-hydroxy prostaglandin of formula (11) into an organic fulsonyloxy group.

According to this invention, the 7-organic sulfonyloxy prostaglandin is then treated with a basic compound.

This second reaction can generally be carried out at the same temperature using the same basic compound as in the first reaction.

The first and second reactions in the process of this invention may be carried out such that after the 7-organic sulfonyloxy prostagrandin formed in the first reaction is isolated, the second reaction is performed; or that without isolating the 7-organic sulfonyloxy prostaglandin formed in the first reaction, the second reaction is performed in the same reaction system or after optionally adding a fresh supply of the basic compound to the reaction system.

According to this invention, the resulting

compound is generally separated from the reaction system, and then as required, the protective groups ($R^{41}$, $R^{51}$) for the hydroxyl groups are eliminated (deprotection) and/or the group $-COOR^{11}$ is converted to a carboxyl group by hydrolysis, or to a salt by salt-forming reaction.

Elimination of the protective groups for the hydroxyl groups can be carried out as follows: When the protective groups are groups forming acetal linkages with oxygen atoms, the deprotection may be conveniently carried out in a reaction solvent such as water, tetrahydrofuran, ethyl ether, dioxane, acetone or acetonitrile using acetic acid, pyridinium p-toluenesulfonate, a cation exchange resin, etc. as a catalyst. The reaction is carried out usually at -20°C to +80°C for a period of 10 minutes to 3 days. When the protective groups are tri($C_{1-7}$ hydrocarbon) silyl groups, the deprotection may be carried out at the same temperature for the same period of time as above in the above-exemplified reaction solvent in the presence of acetic acid, hydrogen fluoride, tetrabutyl ammonium fluoride, or cesium fluoride, preferably acetic acid and hydrogen fluoride.

Hydrolysis of the ester group ($-COOR^{11}$) may be carried out, for example, by treating the resulting compound, with or without prior deprotection, with an enzyme such as lipase in water or a water-containing solvent at a temperature in the range of 0°C to 40°C for a period of 10 minutes to 24 hours.

The salt-forming reaction is carried out by neutralizing the carboxylic acid with a nearly equivalent amount of a basic compound such as sodium hydroxide, potassium hydroxide, sodium carbonate, ammonia, or trimethylamine in a usual manner.

Thus, there are obtained the 5-thiaprostaglandin presented by the formula (1)-a-1.

The 5-thiaprostaglandin (when the symbol ===== between the 7- and 8-positions indicates a single bond) of the following formula (1)-a-2

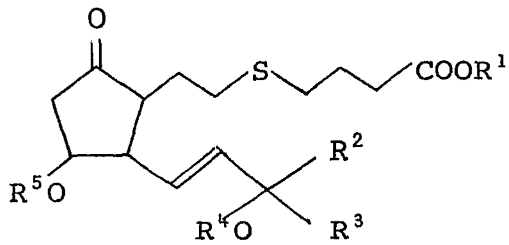

(1)-a-2

wherein $R^1$, $R^2$, $R^3$, $R^4$ and $R^5$ are defined as above,

are produced in accordance with the present invention by selectively reducing the 5-thiaprostaglandin represented by the following formula (1)-a-1

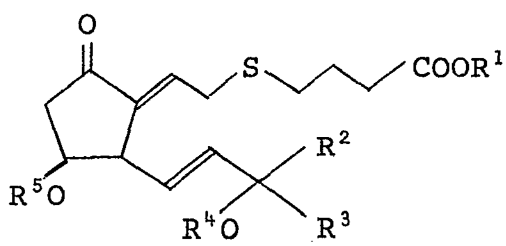

(1)-a-1

wherein $R^1$, $R^2$, $R^3$, $R^4$ and $R^5$ are defined as above,

and if required, subjecting the reaction product to a reaction of removing the protective group, a reaction of hydrolysis, or a salt-forming reaction.

According to the process of this invention, the selective reduction of the 5-thiaprostaglandin of formula (1)-a-1 is carried out preferably by using metallic zinc or a reducing agent containing metallic zinc or by catalytic reduction in the presence of a Raney nickel catalyst, or by using

organic tin hydride. When metallic zinc or the reducing agent containing metallic zinc is used, the reaction proceeds advantageously in the presence of a carboxylic acid such as glacial acetic acid, propionic acid or trichloroacetic acid. The metallic zinc or the reducing agent containing it is used in an amount of about 1 to about 100 moles, preferably about 5 to about 50 moles, as metallic zinc. The carboxylic acid is used in an amount of 1 to 20 moles, preferably 2 to 10 moles, per mole of metallic zinc in the reducing agent.

The reaction is desirably carried out in the presence of a solvent, for example alcohols such as methanol, ethanol, isopropanol or butanol; acetic acid, dimethoxyethane, dimethylformamide dimethyl sulfoxide, or mixtures of these. The alcohols, especially isopropanol, are preferred as the solvent. The catalytic reduction with Raney nickel, on the other hand, is carried out in an atmosphere of hydrogen. The Raney nickel catalyst is used in an amount of 0.01 to 0.2 part by weight, preferably 0.05 to 0.1 part by weight, per part by weight of the 5-thiaprostaglandin of formula (1)-a-1. To cause the reaction to proceed smoothly, a solvent is preferably used. Examples of the solvent are alcohols such as methanol, ethanol and butanol, ethers such as dimethoxyethane and tetrahydrofuran, dimethylformamide, and mixtures of these. Methanol is preferred.

The selective reduction may be carried out by using organic tin hydride. Examples of the organic tin hydride are tri-alkyl tin hydride such as tri-butyl tin hydride and tri-ethyl tin hydride, and tri-phenyl tin hydride. The organic tin hydride is used in an amount of 1 to 10,000 moles, preferably 1 to 1,000 moles, per mole of 5-thiaprostaglandin of formula (1)-a-1.

The reaction can be carried out in the presence of a solvent, for example alchols such as methanol, ethanol or propanol, but the reaction is preferably carried out without using a solvent. The reaction may be carried out in the presence of a radical generator such as α,α'-azobisisobutyronitrile or bis-t-butylperoxide. The radical generator is used in an amount of 0.01 to 10 moles, preferably 0.1 to 1 moles per moles of 5-thiaprostaglandin of formula (1)-a-1.

In the aforesaid selective reduction, the double bond between the 7- and 8-positions is stereoselectively reduced so that a product in which the α-chain and β-chain are trans to each other is formed in a major proportion. Accordingly, if the above selective reduction is carried out by using natural-type 5-thiaprostaglandin compounds of (the β-chain is of β-configuration and the group $-OR^{41}$ is of α-configuration) which are embraced by formula (1)-a-1, the corresponding 5-thiaprostaglandin compounds of the natural-type can be selectively obtained.

The resulting 5-thiaprostaglandin is separated from the reaction system, and, as required, in the same way as described hereinabove, the protective groups for the hydroxyl groups are eliminated, and/or the ester group in $-COOR^1$ is converted to a carboxyl group by hydrolysis, or to a salt by salt-forming reaction.

Thus, there are obtained the 5-thiaprostaglandin represented by the formula (1)-a-2.

The 5-thiaprostaglandin represented by the formula (1)-a-2 can also be prepared according to the following method.

That is way, the 5-thiaprostaglandin of the formula (1)-a-2 can also be produced in accordance with the present invention by reacting a α-vinylketone of the following formula (111)

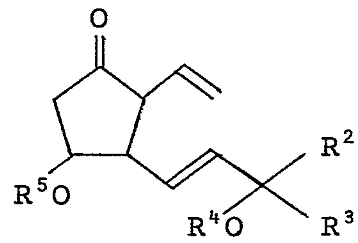

(111)

wherein $R^2$, $R^3$, $R^4$ and $R^5$ are defined as above,

and a thiol compound of the following formula

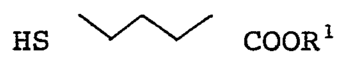

(1V)

wherein $R^1$ is defined as above,

in the presence of a radical generator, and if required, subjecting the reaction product to a reaction of removing the protective group, a reaction of hydrolysis, or a salt-forming reaction.

The α-vinylketon of formula (111) can be produced as shown in the following reaction scheme in accordance with the same process as described in J. Amer. Chem. Soc., 7950, 102, (1980).

<u>reaction scheme</u>

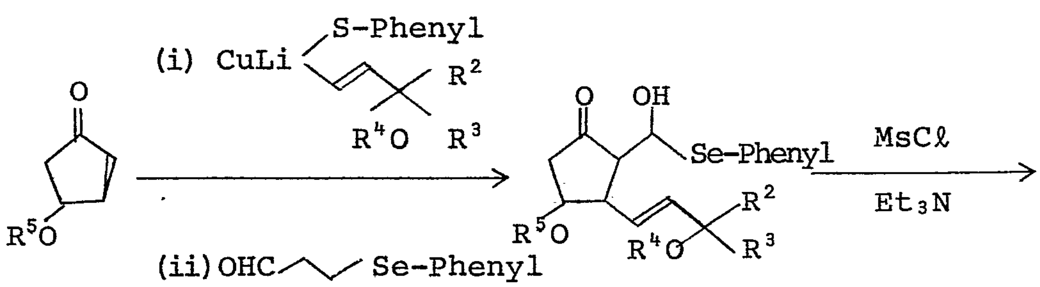

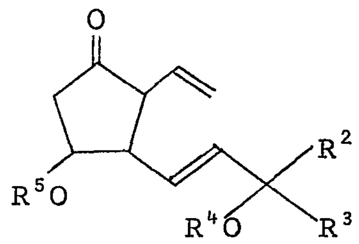

The thiol compound of formula (lV) is known.

The reaction between the α-vinylketone of formula (lll) and the thiol compound of formula (lV) is carried out in the presence of a radical generator, or under ultraviolet irradiation.

Exemplary of these radical generators are t-butyl hydroperoxide, di-t-butyl peroxide, cumene hydroperoxide, benzoyl peroxide, lauroyl peroxide and α,α'-azobisisobutyronitrile.

Ultraviolet light used has a wavelength in the region of about 200 nm to about 400 nm.

Preferably, the reaction is carried out in the presence of an inert organic solvent. Examples of suitable inert organic solvents are ethers, aromatic hydrocarbons and halogenated hydrocarbons. Examples of the ethers include diethylether, tetrahydrofuran, dioxane and dimethoxyethane. Examples of the aromatic hydrocarbons include benzene, toluene and xylene. Examples of the halogenated hydrocarbons include chloroform, tetrachloromethane and dichloromethane.

When the reaction is carried out in the presence of the radical generator, the reaction temperature is usually -30°C to +200°C, preferably 0°C to 100°C. When the reaction is carried out under ultraviolet irradiation,the reaction temperature is usually from -100°C to +50°C, preferably from -78°C to +30°C.

Stoichiometrically, the α-vinylketone of formula (lll) and the thiol compound of formula

(lV) in equimolar proportions. The thiol compound is used in an amount of usually about 0.9 to about 10 moles, preferably 1 to 3 moles, per mole of the α-vinylketone.

The reaction is carried out conveniently in an atmosphere of an inert gas such as nitrogen and argon.

The resulting 5-thiaprostaglandin is separated from the reaction system, and, as required as, in the same way as described hereinabove, the reaction product may be subjecting to elimination, hydrolysis, or salt-forming reaction.

Thus, there is obtained the 5-thiaprostaglandin of the formula (l)-a-2.

The 5-thiaprostaglandin of the following formula (l)-b

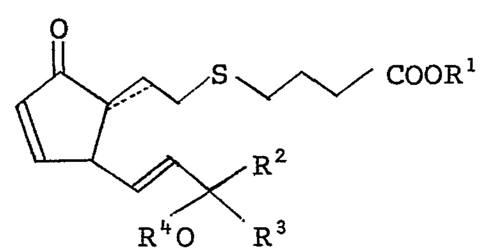

(l)-b

wherein $R^1$, $R^2$, $R^3$ and $R^4$ are defined as above,

are produced in accordance with the present invention by subjecting a 5-thiaprostaglandin of the following formula (V)

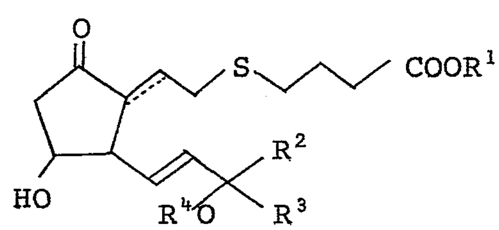

(V)

wherein $R^1$ and $R^4$ are defined as above, $R^3$ represents an unsubstituted $C_3$-$C_8$ linear or branched alkyl group, a substituted $C_1$-$C_5$ linear or branched alkyl group substituted by a substituent selected from phenyl, phenoxy, $C_1$-$C_6$ alkoxy, $C_5$-$C_6$ cycloalkyl, which substituent may be substituted, or a substituted or unsubstituted alicyclic group,

to dehydration reaction, and if required, subjecting the reaction product to a reaction of removing the protective group, a reaction of hydrolysis, or a salt-forming reaction.

The 5-thiaprostaglandin of the formula (V) is obtained by the aforementioned process in the present invention.

The dehydration reaction of 5-thiaprostaglandin of formula (V) is preferably carried out in the presence of a dehydrating agent. Examples of the dehydrating agent include inorganic acids such as hydrochloric acid, hydrobromic acid, hydrofluoric acid and phosphoric acid, organic carboxylic acids such as propionic acid, oxalic acid, citric acid and maleic acid, and organic sulfonic acids such as methanesulfonic acid, ethanesulfonic acid, benzenesulfonic acid and p-toluenesulfonic acid. Of these, the inorganic acids and organic carboxylic acids are preferred. The amount of the dehydrating agent used is preferably 0.5 to 100 moles, especially preferably 1 to 50 moles, per mole of the 5-thiaprostalandin. As a reaction solvent, there may be used an ether such as tetrahydrofuran, dioxane, dimethoxyethane or diethyl ether, an alcohol such as methanol or ethanol, dimethyl sulfoxide, dimethylformamide, hexamethylphosphoric triamide, acetonitrile, and water, either singly or in combination with each other.

The reaction temperature is preferably 0 to 80°C, especially preferably 10 to 50°C.

The reaction time varies depending upon the starting compound, the dehydrating agent and the reaction solvent used in the reaction. Usually, it is 10 minutes to 10 days, preferably 20 minutes to 5 days.

It is also possible to subject the 5-thioprostalandin of formula (1)-a in which the 11-position hydroxyl group is protected by a protective group to a deprotection reaction using a deprotecting aid such as acetic acid thereby removing the protective group at the 11-position and forming the 5-thiaprostaglandin of formula (V) in situ, and subsequently to subject this reaction mixture to the dehydration reaction in accordance with this invention.

According to the dehydration reaction of this invention, a corresponding compound (final desired compound) having a double bond formed between the 10- and 11-positions in formula (V) as a result of removal of the 11-position hydroxyl group is formed.

As required, the reaction product may be subjected to elimination, hydrolysis, or salt-forming reaction.

Thus, the 5-thiaprostaglandin of formula (1)-b is obtained.

Investigations of the present inventors have shown that a 5-thiaprostaglandin expressed by the following formula

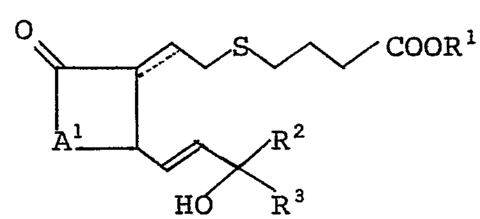

(1)'

wherein $R^1$ and the symbol ┈┈┈ are defined as above, $A^1$ represents $-CH_2-\underset{OH}{CH}-$ or $-CH=CH-$ (cis), $R^2$ represents a hydrogen atom or a methyl group, $R^3$ represents an unsubstituted $C_3-C_8$ linear or branched alkyl group, a substituted $C_1-C_5$ linear or branched alkyl group substituted by a substituent selected from phenyl, phenoxy, $C_1-C_6$ alkoxy, $C_5-C_6$ cycloalkyl, which substituent may be substituted, or a substituted or unsubstituted alicyclic group,

have especially superior pharmacological activities, for example an anti-ulcer, antitumor, blood pressure lowering activity, or platelet aggregation inhibiting activity.

Thus, the present invention provides a pharmaceutical composition comprising as an active ingredient the 5-thiaprostaglandin of formula (1'), and a pharmaceutically acceptable carrier.

The 5-thiaprostaglandin of formula (1') has superior pharmacological activities, and better selective activity than natural $PGE_1$.

The 5-thiaprostaglandin of the present invention can be administered orally or parenterally such as percutaneously, subcutaneously, intramuscularly, intravenously, vaginally, reactally, etc.

As the form of preparations for oral administration, tablets, pills, granules, powders, solutions, suspensions, and capsules, for instance, may be mentioned.

Tablets can be formed according to the ordinary method by use of such excipients as lactose, starch, polyvinyl pyrrolidone, calcium carbonate, microcrystalline cellulose, silicic acid, etc., such binders as carboxymethyl cellulose, methyl cellulose,

potassium phosphate, etc., such disintegrators as sodium alginate, sodium hydrogencarbonate, sodium lauryl sulfate, monoglyceride stearate, etc., such moisteners as glycerin, etc., such adsorbents as kaolin, colloidal cilicic acid, etc., and such lubricants as refined talc, powdered boric acid, etc. Pills, powders, granules can also be formed according to the ordinary method by use of the abovementioned excipients and other additive agents.

Solutions and suspensions can be prepared according to the ordinary method by use of such glyceryl esters as tricaprylin, triacetin, etc., such vegetable fats and oils as cocount oil, fractionated coconut oil, etc., purified water, such alcohols as ethanol, etc. Capsules can be prepared by filling capsules made from gelatin, etc. with granules, powders, and solutions.

As the form of preparations for percutaneous administration, ointments and creams may be mentioned. Ointments can be prepared according to the ordinary method by use of such vegetable fats and oils as castor oil, olive oil, etc. and vaseline, etc. and creams can be prepared likewise by use of fats and oils and such emulsifying agents as diethylene glycol, sorbitan mono fatty acid ester, etc.

As the form of preparations for subcutaneous, intramuscular, and intravenous administration, there are injections prepared in the form of an aqueous or non-aqueous solution or of a suspension. Non-aqueous solutions and suspensions are usually prepared by use of, for instance, propylene glycol, polyethylene glycol, olive oil, ethyl oleate, etc., with antiseptics and/or stabilizers added thereto as case may require. Injections are sterilized by filtration through a bacterial filter or by mixing of a germicide effected appropriately.

As the form of preparations for vaginal administration, film preparations prepared by use of polyvinyl pyrrolidone, hydroxy propyl cellulose, etc. or ointments may be mentioned.

As the form of preparations for rectal administration such ordinary suppositories as gelatin soft capsules, etc. may be mentioned.

The 5-thiaprostaglandin which are the active compound of the present invention can also be allowed to be contained in the preparations as the inclusion compounds of α-, β-, or γ-cyclodextrins or their methylated cyclodextrins.

The active compound of this invention is administered, either singly or as the aforesaid pharmaceutical composition, or as a medicament in unit dosage form.

The dose of the active compound varies depending upon the type of the active compound, the subject to which it is administered, the conditions, age, sex, and body weight of the subject, or the route of administration. Usually it can be administered in a dose of about 0.01 μg to about 20 mg/kg of body weight/day. The dose may be administered once a day or in several portions, for example 2 to 6 times a day.

The following Examples illustrated the present invention in greater detail.

Referential Example 1

Synthesis of 5-thia-$\Delta^7$-prostaglandin $E_1$ methyl ester 11,15-bis(t-butyldimethylsilyl)ether and its 15-epimer:-

(i) 6.07g (16.5 mmol) of 3(S)-t-butyldimethylsilyl-oxy-1-iodo-1-octene was dissolved in 30 mℓ of anhydrous ether, and the solution was cooled to -78°C. 16.5mℓ (33 mmol) of t-butyl lithium was added, and the mixture was stirred at -78°C for one hour. To the solution was added a solution of 2.15 g (16.5 mmol) of 1-pentynyl copper (1) and 5.4 g (33 mmol) of hexamethyl-phosphoric triamide in 25 mℓ of anhydrous ether, and the resulting mixture was stirred at -78°C for one hour. To the resulting mixture was added a solution of 3.18 g (15 mmol) of 4-t-butyldimethylsilyoxy-2-cyclopentenone in 20 mℓ of anhydrous ether. The mixture was stirred at -78°C for 30 minutes and then at -50°C for 10 minutes. A solution cooled to -50°C of 2.9 g (16.5 mmol) of 7-oxo-5-thiaheptanoic acid methyl ester in 10 mℓ of anhydrous ether was added to the resulting solution, and the mixture was stirred at -50°C to -40°C for 40 minutes. The reaction mixture was poured into an acetic acid/sodium acetate buffer adjusted to pH 4, and thereafter extracted with hexane. The extraction was dried over anhydrous magnesium sulfate, filtered and concentrated. The residue was chromatographed on a silica gel column to give 5.2 g (yield 55%) of 7-hydroxy-5-thia-prostagandin $E_1$ methyl ester 11,15-bis(t-butyldimethylsilyl)ether and its 15-epimer.

TLC ; Rf =
0.45 (hexane : ethyl acetate = 3 : 1)

IR ($cm^{-1}$, neat) ;
3500, 2950, 2880, 1740, 1460, 1440, 1360, 1250.

NMR (δ ppm in $CDCl_3$);

0.9 (s. 18H), 0.9 - 1.7 (m. 11H),
1.7 - 3.1 (m. 10H), 3.7 (s. 3H),
3.7 - 4.4 (m. 3H), 5.65 (m. 2H).

(ii) 1.25 mg (1.98 mmol) of 7-hydroxy-5-thiaprostalandin $E_1$ methyl ester 11,15-bis(t-butyldimethylsilyl) ether and its 15-epimer was dissolved in 10 mℓ of anhydrous dichloromethane, and 1.65 g (13.5 mmol) of 4-dimethylaminopyridine was added. With stirring under ice cooling, 0.5 mℓ (6.7 mmol) of methanesulfonyl chloride was added. The mixture was stirred at 0°C for 5 hours and then at room temperature for additional one hour. Water was added, and the mixture was extracted with dichloromethane, dried over anhydrous magnesium sulfate, filtered, and concentrated. The residue was chromatographed on a silica gel column to give 5-thia-$\Delta^7$-prostaglandin $E_1$ methyl ester 11,15-bis(t-butyldimethylsilyl) ether and its 15-epimer. 590 mg (yield 49%) of (7E)-type, and 150 mg (yield 12%) of (7Z)-type was obtained.

TLC ; 7E-type, Rf = 0.655 (hexane : ethyl acetate = 3 : 1)

7Z-type, Rf = 0.68 (hexane : ethyl acetate = 3 : 1)

7E-type

IR ($cm^{-1}$, neat) :

2980, 2870, 1740, 1640, 1460,
1360, 1255

NMR (δ ppm in $CDCl_3$) :

0.99 (s. 18H), 0.7 - 2.1 (m. 13H),
2.1 - 2.8 (m. 6H), 3.0 - 3.5 (m. 3H),
3.70 (s. 3H), 3.9 - 4.3 (m. 2H),
5.55 (m. 2H), 6.8 (dt, 1H, J = 7.5, 2.0)

7Z-type

IR ($cm^{-1}$, neat) :

2980, 2870, 1740, 1450, 1255

NMR ($\delta$ ppm in $CDCl_3$) :

0.9 (s. 18H), 0.7 - 2.1 (m. 13H),
2.21 - 2.7 (m. 6H), 3.0 - 3.6 (m. 3H),
3.7 (s. 3H), 3.7 - 4.35 (m. 2H),
5.3 - 5.9 (m. 1H), 5.55 (m. 2H)

Referential Example 2

<u>Synthesis of (7E)-$\Delta^7$-5-thiaprostaglandin $E_1$ methyl ester and its 15-epimer:-</u>

213 mg (0.35 mmol) of (7E)-$\Delta^7$-5-thiaprostaglandin $E_1$ methyl ester 11, 15-bis(t-butyldimethylsilyl)ether and its 15-epimer was dissolved in 10 m$\ell$ of acetonitrile, and to the solution was added 0.5 m$\ell$ of 40% hydrofluoric acid.

The mixture was stirred at room temperature for 30 minutes. By adding a aqueous solution of sodium hydrogen carbonate, the reaction was over. The resulting mixture was extracted with ethyl acetate, dried, filtered, and concentrated. The residue was purified by silica gen thin-layer chromatography to give 100 mg (yield 75%) of (7E)-$\Delta^7$-5-thiaprostaglandin $E_1$ methyl ester and its 15 epimer.

TLC ; Rf =

0.45 (hexane : ethyl acetate = 1 : 4)

IR ($cm^{-1}$, neat) :

3400, 2770, 2750, 2670, 1720,
1640, 1440, 1240

NMR ($\delta$ ppm in $CDCl_3$) :

0.9 (m. 3H), 0.7 - 2.0 (m. 8H),
1.9 (dd. 2H, J = 7), 2.2 - 3.0 (m. 8H),
3.25 (d. 2H, J = 8), 3.5 (m. 1H),
3.7 (s. 3H), 4.2 (m. 2H), 5.65 (m. 2H),
6.8 (dd. J = 8.0, 1.5)

Referential Example 3

Synthesis of (7E)-$\Delta^7$-5-thiaprostaglandin $E_1$:-

54 mg (0.14 mmol) of (7E)-$\Delta^7$-5-thiaprostaglandin $E_1$ methyl ester was dissolved in 0.8 mℓ of acetone, and 8 mℓ of a phosphate buffer (pH 8) was added. Thereafter 80 μℓ of a suspension of pig liver esterase was added, and the mixture was stirred at room temperature for 4 hours.

With ice cooling, the mixture was adjusted to pH 3.5 with dilute hydrochloric acid. The mixture was extracted with ethyl acetate, dried, filtered and concentrated. The residue was purified by silica gel thin-layer chromatography to give (7E)-$\Delta^7$-5-thiaprostaglandin $E_1$.

NMR (δ ppm in $CDCl_3$) :

0.7 - 2.0 (m. 11H), 1.9 (dd. 2H, J = 7),
2.2 - 3.0 (m. 9H), 3.25 (d. 2H, J = 8),
3.5 (m. 1H), 4.2 (m. 2H), 5.6 (m. 2H),
6.8 (dd. J = 8.0, 1.5)

Example 1

Synthesis of (7E)-$\Delta^7$-5-thiaprostaglandin $A_1$ methyl ester and its 12-epimer:-

121 mg (0.2 mmol) of (7E)-$\Delta^7$-5-thiaprostaglandin $E_1$ methyl ester 11,15-bis(t-butyldimethylsilyl)ether and its 15-epimer was dissolved in 10 mℓ of acetonitrile, and 0.5 mℓ of 40% hydrogen fluoric acid was added. The mixture was stirred at 40°C for 4 hours. Then the reaction mixture was cooled to room temperature. By adding a aqueous solution of sodium hydrogen carbonate, the reaction was over. The mixture was extracted with ethyl acetate, dried, filtered and concentrated. The residue was purified by silica gel thin-layer chromatography using hexane-ethyl acetate (1 : 3) as an eluent to give 23 mg

(yield 32%) of (7E)-$\Delta^7$-5-thiaprostaglandin $A_1$ methyl ester (Rf = 0.5) and 24 mg (yield 33%) of its 12-epimer (Rf = 0.57).

(7E)-$\Delta^7$-5-thiaprostaglandin $A_1$ methyl ester:-

IR ($cm^{-1}$, neat) :
3450, 2950, 2870, 1735, 1700,
1645, 1580, 1435

NMR ($\delta$ ppm in $CDCl_3$) :
0.8 - 2.2 (m. 14H), 2.2 - 2.8 (m. 4H),
3.3 (d. 2H, J = 8.0), 3.7 (s. 3H),
4.0 - 4.35 (m. 2H),
5.5 (dd. 1H, J = 15.0, 6.0),
5.9 (dd. 1H, J = 15.0, 6.0),
6.4 (dd. 1H, J = 6.0, 2.0),
6.7 (t. 1H, J = 8.0),
7.5 (dd. 1H, J = 6.0, 3.0)

Mass (20 eV, m/e) :
366 ($M^+$), 348, 215, 134, 120,
101 (100%)

12-epimer

IR ($cm^{-1}$, neat) :
3450, 2950, 2870, 1735, 1700,
1645, 1580, 1435

NMR ($\delta$ ppm in $CDCl_3$) :
0.8 - 2.2 (m. 14H), 2.2 - 2.8 (m. 4H),
3.35 (d. 2H, J = 8.0), 3.7 (s. 3H),
3.9 - 4.4 (m. 2H),
5.4 (dd. 1H, J = 16.0, 6.0),
5.85 (dd. 1H, J = 16.0, 6.0),
6.4 (dd. 1H, J = 6.0, 2.0),
6.7 (t. 1H, J = 8.0),
7.4 (dd. 1H, J = 6.0, 3.0)

Example 2

Synthesis of 16,17,18,19,20-pentanor-15-cyclohexyl -5-thia-$\Delta^7$-prostaglandin $E_1$ methyl ester 11,15-bis(t-butyldimethylsilyl)ether:-

560 mg (0.85 mmol) of 16,17,18,19,20-pentanor-15-cyclohexyl-7-hydroxy-5-thiaprostaglandin $E_1$ methyl ester 11,15-bis(t-butyldimethylsilyl)ether obtained by the same operation as in (i) of Referential Example 1 was dissolved in 5 mℓ of anhydrous dichloromethane, and 530 mg (4.35 mmol) of 4-dimethylaminopyridine was added. The mixture was cooled over an ice bath, and 170 μℓ (2.2 mmol) of methanesulfonyl chloride was added. The mixture was stirred at 0°C for 2 hours and then at room temperature for 3 hours.

To the reaction mixture was added water, and the mixture was extracted with dichloromethane, dried over anhydrous magnesium sulfate, filtered and concentrated. The residue was chromatographed over a silica gel column to give 315 mg (yield 58%) of 16,17,18,19,20-pentanor-15-cyclohexyl-5-thia-$\Delta^7$-prostaglandin $E_1$ methyl ester 11,15-bis(t-butyldimethylsilyl)ether[mixture of (7E) and (7Z) (4:1)].

IR ($cm^{-1}$, neat) :

2970, 2860, 1740, 1640, 1455,
1355, 1260

NMR (δ ppm in $CDCl_3$) :

0.9 (s. 18H), 0.9 - 2.2 (m. 13H),
2.2 - 2.7 (m. 6H), 3.0 - 3.65 (m. 3H),
3.65 (s. 3H), 3.7 - 4.4 (m. 2H),
5.2 - 5.9 (m. 0.2H, 7Z-type), 5.5 (m. 2H),
6.9 (t. 0.8H, J = 8, 7E-type)

Example 3

Synthesis of 16,17,18,19,20-pentanor-15-cyclohexyl-7(E)-$\Delta^7$-5-thiaprostaglandin $A_1$ methyl ester:-

102 mg (0.26 mmol) of 16,17,18,19,20-pentanor-15-cyclohexyl-7(E)-$\Delta^7$-5-thiaprostaglandin $E_1$ methyl ester was dissolved in 2 mℓ of tetrahydrofuran, and a aqueous solution (0.5N) of hydrochloric acid was added. The mixture was stirred at 40°C for 3 hours. By adding a aqueous solution of sodium hydrogen carbonate, the reaction was over. The reaction mixture was extracted, dried, filtered, and concentrated. The residue was purified by silica gel thin-layer chromatography to give 55 mg (yield 56%) of 16,17,18,19,20-pentanor-15-cyclohexyl-7(E)-$\Delta^7$-5-thiaprostaglandin $A_1$ methyl ester.

TLC : Rf = 0.5 (hexane : ethyl acetate = 1 : 3)

IR ($cm^{-1}$, neat) :

2960, 2860, 1740, 1700, 1645, 1575

NMR (δ ppm in $CDCl_3$

0.8 - 2.2 (m. 14H), 2.2 - 2.7 (m. 4H),
3.3 (d. 2H, J = 8.0), 3.65 (s. 3H),
3.9 -4.3 (m. 2H),
5.5 (dd. 1H, J = 15.0, 6.0),
5.9 (dd. 1H, J = 15.0, 6.0),
6.4 (dd. 1H, J = 6.0, 2.0),
6.7 (t. 1H, J = 8.0),
7.5 (dd. 1H, J = 6.0, 3.0)

Example 4

Synthesis of 17(S),20-dimethyl-5-thia-$\Delta^7$-prostaglandin $E_1$ methyl ester 11,15-bis(t-butyldimethylsilyl)ether:-

120 mg (0.18 mmol) of 7-hydroxy-17(S),20-

dimethyl-5-thiaprostaglandin $E_1$ methyl ester 11,15-bis(t-butyldimethylsilyl)ether obtained by the same operation as in (i) of Referential Example 1 was dissolved in 2 mℓ of anhydrous dichloromethane, and 110 mg (0.9 mmol) of 4-dimethylaminopyridine was added.

The mixture was cooled over an ice bath, and 30 μℓ (0.4 mmol) of methanesulfonyl chloride was added. The mixture was stirred at 0°C for one hour and then at room temperature for 5 hours.

To the reaction mixture was added water, and the mixture was extracted with dichloromethane, dried over anhydrous magnesium sulfate, filatered, and concentrated. The residue was purified by silica gel then-layer chromatography to give 76 mg (yield 66%) of 17(S),20-dimethyl-5-thia-$\Delta^7$-prostaglandin $E_1$ methyl ester 11,15-bis(t-butyldimethylsilyl)ether[mixture of (7E) and (7Z) (4:1)].

IR ($cm^{-1}$, neat) :

2980, 2860, 1740, 1640, 1460, 1360, 1255

NMR (δ ppm in $CDCℓ_3$) :

0.9 (s. 18H), 0.7 - 2.0 (m. 17H), 2.0 - 2.7 (m. 6H), 3.0 - 3.7 (m. 3H), 3.70 (s. 3H), 3.7 - 4.5 (m. 2H), 5.2 - 5.9 (m. 0.2H, 7Z-type), 5.6 (m. 2H), 6.9 (t. 0.8H, J = 8, 7E-type)

Example 5

Synthesis of 17(R),20-dimethyl-$\Delta^7$-5-thiaprostaglandin $E_1$ methyl ester 11,15-bis(t-butyldimethylsylyl)ether:-

(i) 17(R),20-dimethyl-7-hydroxy-5-thiaprostaglandin $E_1$ methyl ester 11,15-bis(t-butyldimethylsilyl)ether was obtained in the same way as in (i) of Referential Example 1 from (4R)-4-t-butyldimethylsilyloxy-2-cyclopentanone, (3S, 5R)-3-t-butyldimethylsilyloxy-1-iodo-5-methyl-1-nonene and 7-oxo-5-thia-heptanoic acid methyl.

Rf = 0.32 (hexane : ethyl acetate = 4 : 1)

NMR ($\delta$ ppm in $CDCl_3$) :

0 - 0.2 (12H, m), 0.87 (18H, s),
0.7 - 1.0 (6H, m), 1.0 - 3.3 (22H, m),
3.59 (3H, m), 3.6 - 4.3 (3H, m),
5.3 - 5.7 (2H, m)

(ii) 17(R),20-dimethyl-$\Delta^7$-5-thiaprostaglandin $E_1$ methyl ester 11,15-bis(t-butyldimethylsilyl)ether was obtained in the same way as in (ii) of Referential Example 1.

Rf = 0.48, 0.45 (hexane : ethyl acetate = 5 : 1)

NMR ($\delta$ ppm in $CDCl_3$) :

0 - 0.2 (12H, m), 0.86 (18H, s),
0.7 - 1.0 (6H, m), 1.0 - 2.15 (12H, m),
2.15 - 2.7 (6H, m), 2.95 - 3.25 (2H, m),
3.3 - 3.5 (1H, m), 3.58 (2H, S),
3.8 - 4.3 (2H, m), 5.3 - 5.6 (2H, m),
6.62 (1H, dt. J = 7.8, 1.7HZ)

Example 6

<u>Synthesis of 17(R),20-dimethyl-5-thiaprostaglandin $E_1$ methyl ester 11,15-bis(t-butyldimethylsilyl)ether and its 8-epimer:-</u>

350 mg (546 μmol) of 17(R),20-dimethyl-$\Delta^7$-5-thiaprostaglandin $E_1$ methyl ester 11,15-bis(t-butyldimethylsilyl)ether was dissolved in 3 mℓ of tri-n-butyl tinhydride, and 20 mg of bis-t-butylperoxide was added. The mixture was stirred at 100°C for 15 minutes and then at 110°C for 40 minutes. The resulting mixture was cooled to room temperature, and then purified by silica gel column chromatography using cyclehexane-ethyl acetate (1:0 → 15:1) as an eluent to give 167 mg (yield 48%)

of 17(R),20-dimethyl-5-thiaprostaglandin $E_1$ methyl ester 11,15-bis(t-butyldimethylsilyl)ether and 46 mg (yield 13%) of its 8-epimer.

17(R),20-dimethyl-5-thiaprostaglandin $E_1$ methyl ester 11,15-bis(t-butyldimethylsilyl)ether:-

Rf = 0.45 (hexane : ethyl acetate = 5 : 1)

IR ($cm^{-1}$, neat) : 1737

NMR (δ ppm in $CDCl_3$) :

0 - 0.2 (12H, m), 0.87 (18H, S),
0.7 - 1.0 (6H, m), 1.0 - 2.1 (13H, m),
2.1 - 2.8 (10H, m), 3.58 (3H, t),
3.7 - 4.3 (2H, m), 5.3 - 5.7 (2H, m)

8-epimer:-

Rf = 0.52 (hexane : ethyl acetate = 5 : 1)

IR ($cm^{-1}$, neat) : 1737

NMR (δ ppm in $CDCl_3$) :

0 - 0.2 (12H, m), 0.87 (18H, s),
0.7 - 1.0 (6H, m), 1.0 - 2.1 (13H, m),
2.1 - 3.0 (10H, m), 3.57 (3H, s),
3.7 - 4.3 (3H, m), 4.99 (1H, dd. J = 15.0, 9.0Hz), 5.47 (1H, dd. J = 15.0, 6.8Hz)

Example 7

Synthesis of 17(R),20-dimethyl-5-thiaprostaglandin $E_1$ methyl ester:-

167 mg (0.20 mmol) of 17(R),20-dimethyl-5-thiaprostaglandin $E_1$ methyl ester 11,15-bis(t-butyldimethylsilyl)ether was dissolved in 10 mℓ of acetonitrile, and 1 mℓ of pyridine and 2 mℓ of a hydrogen fluoridepyridine. The solution was stirred at room temperature for 15 minutes. After adding a saturated aqueous solution of sodium hydrogen carbonate, the mixture was extracted with ethyl acetate. The organic layers were combined, washed with 0.5N hydrochloric acid, a saturated aqueous solution of sodium hydrogen carbonate and

then a saturated aqueous solution of sodium chloride, dried, and concentrated. The residue was chromatographed on a column of silica gel using cyclohexane-ether-methanol (1 : 1 : 0.01 → 1 : 3 : 0.01) to give 85 mg (yield) 79%) of 17(R),20-dimethyl-5-thiaprostaglandin $E_1$ methyl ester.

Rf = 0.31 (hexane : ethyl acetate = 1 : 4)

IR ($cm^{-1}$, neat) : 3390, 1733

NMR (δ ppm in $CDCl_3$) :

0.7 - 1.1 (6H, m), 1.1 - 3.0 (23H, m),
3.61 (3H, s), 3.7 - 4.4 (3H, m),
5.4 - 5.7 (2H, m)

Referential Example 4

Synthesis of 5-thiaprostaglandin $E_1$ methyl ester and its 8,11,12-epimer:-

(i) 736 mg (2 mmol) of 3(S)-t-butyldimethylsilyloxy-1-iodo-1-octene was dissolved in 4 mℓ of anhydrous ether, and the solution was cooled to -78°C. Pentane solution of 2.0 mℓ (4 mmol) of t-butyllithium was added. And 4 mℓ of anhydrous ether solution of 380 mg ( 2 mmol) of copper iodide and 890 mg (4.4 mmol) of tri-butyl phosphine was add, and the mixture was stirred at -78°C for one hour. To the resulting mixture was added 385 mg (1.81 mmol) of 4-t-butyldimethylsilyloxy-2-cyclopentenone, and the mixture was stirred at -78°C for one hour and then at 40°C for 10 minutes. To the resulting mixture was added 4 mℓ of anhydrous ether solution of 458 mg (2.3 mmol) of phenylselenoacetoaldehyde, the mixture was stirred for one hour.

By adding an aqueous solution of ammonium chloride, the reaction was over. The mixture was extracted with ether, dried, filtered and concentrated. The residue was purified by silica gel column chromatography using ethyl acetate-hexane

(1 : 8) as an eluent to give 545 mg (yield 46%) of 2-(1-phenylseleno-2-hydroxyethyl)-3(S)-t-butyldimethylsilyloxy-1-octynyl)-4-t-butyldimethylsilyloxycyclopentanone.

IR (neat, $cm^{-1}$) :

3460, 2950, 2900, 2870, 1745

1580, 1480, 1475, 1440

NMR (δ ppm in $CDCl_3$) :

0.9 (s, 18H), 1.1 - 1.6 (m, 11H),
2.2 - 3.5 (m, 6H), 3.8 - 4.3 (m, 3H),
5.5 (m, 2H), 7.25 (m, 3H), 7.50 (m, 2H)

(ii) 211 mg (0.32 mmol) of 2-(1-phenylseleno-2-hydroxyethyl)-3-(3(S)-t-butyldimethylsilyloxy-1-octenyl)-4-t-butyldimethylsilyloxycyclopentanone was dissolved in 2 mℓ of tetrachloromethane, and 225 μℓ of triethylamine was added. To the mixture was added 100 μℓ of mesylchloride, and the solution was stirred at room temperature for one hour. The reaction mixture was extracted with ether, dried, filtered and concentrated. The residue was chromatographed on silica gel column using ethyl acetate-hexane (1 : 5) as an eluent to give 62 mg (yield 40%) of 2-vinyl-3-(3(S)-t-butyldimethylsilyloxy-1-octenyl)-4-t-butyldimethylsilyloxy cyclopentanone.

NMR (δ ppm in $CDCl_3$) :

0.9 (s. 8H), 1.1 - 1.6 (m, 4H),
3.8 - 4.3 (m, 2H), 1.5 (m, 1H),
5.3 (m, 1H), 5.6 (m, 3H), 7.3 (m, 3H),
7.6 (m, 2H)

(iii) A mixture of 76 mg of 2-vinyl-3-(3(S)-t-butyldimethylsilyloxycyclopentanone and 150 mg of 4-mercaptobutyric acid methyl ester was stirred in the presence of 15 mg of α,α'-azobis(isobutyronitrile) at 80°C for 4.5 hours. The resulting mixture was purified by silica gel thin-layer chromatography to give 20 mg (yield 21%) of 5-thiaprostaglandin $E_1$ methyl ester 11,15-bis(t-butyldimethylsilyl)ether

and its 8,11,12-epimer.

TLC (benzene : ethyl = 20 : 1) : Rf = 0.65

NMR (δ ppm in $CDCl_3$) :

1.9 (s. 18H), 1.1 - 1.6 (m, 15H), 1.7 - 2.8 (m, 9H), 3.7 (s. 3H), 4.15 (m, 2H), 5.65 (m, 2H)

(iv) 36 mg of 5-thiaprostaglandin $E_1$ methyl ester 11,15-bis(t-butyldimethylsilyl)ether and its 8,11,12-epimer was dissolved in 14 mℓ of acetonitrile, and 0.7 mℓ of 47% hydrofluoric acid was added. The mixture was stirred at room temperature for 30 minutes. By adding a aqueous solution of sodium hydrogen carbonate, the resulting mixture was neutralized, and extracted with ethyl acetate, dried filtered and concentrated. The residue was purified by silica gel thin-layer chromatography using cyclohexane-ethylacetate (1 : 4) as an eluent to give 7 mg (yield 30.9%) of 5-thiaprostaglandin $E_1$ methylester and 7.5 mg (yield 33%) of its 8,11,12-epimer.

<u>5-thiaprostaglandin $E_1$ methyl ester:-</u>

TLC, Rf = 0.15 (cyclohexane : ethyl acetate = 1 : 4)

NMR (δ ppm in $CDCl_3$) :

0.9 (m, 3H), 1.2 - 1.6 (m, 12H), 1.8 - 2.8 (m, 10H), 3.67 (s. 3H), 4.1 - 4.2 (m, 2H), 5.7 (m, 2H),

Mass (70eV, m/e), 368 ($M^+$ - 18), 350.267.235.

<u>8,11,12-epimer:-</u>

TLC, Rf = 0.20 (cyclohexane : ethyl acetate = 1 : 4)

NMR (δ ppm in $CDCl_3$) :

0.2 (m, 3H), 1.1 - 1.6 (m, 12H), 1.8 - 2.9 (m, 10H), 3.57 (s. 3H), 4.1 - 4.25 (m, 2H), 5.7 (m, 2H),

Mass (70eV. m/e) ; 368 ($M^+$ - 18), 350.267.235.

Example 8

Synthesis of 16,17,18,19,20-pentanor-15-cyclopentyl-5-thiaprostaglandin $E_1$ methyl ester:-

A mixture of 65 mg of 2(R)-vinyl-3(S)-(3(S)-t-butyldimethylsilyloxy-3-cyclopentyl-1-propenyl)-4(R)-t-butyldimethylsilyloxycyclopentanone and 150 mg of 4-mercaptobutyric acid methyl ester was stirred in the presence of 15 mg of α,α'-azobis(isobutyronitrile) at 80°C for 5 hours. The resulting mixture was purified by silica gel thin-layer chromatography to give 16 mg (yield 19%) of 16,17,18,19,20-pentanor-15-cyclopentyl-5-thiaprostaglandin $E_1$ methyl ester.

This compound was subjecting to deprotecting reaction in the same as (iv) of Referential Example 4. The reaction mixture was purified by silica gel thin-layer chromatography to give 5.5 mg (yield 55%) of 16,17,18,19,20-pentanor-15-cyclopentyl-5-thia-prostaglandin $E_1$ methyl ester.

TLC, Rf = 0.15 (cyclohexane : ethyl acetate = 1 : 4)

NMR (δ ppm in $CDCl_3$) :

0.9 - 1.8 (m, 13H), 1.8 - 3.0 (m, 10H), 3.65 (3H, s), 3.4 - 4.3 (4H, m), 5.60 (2H, m), Mass (20eV, m/e) ; (366 $M^+$ - 18)

Example 9

Synthesis of 17(S),20-dimethyl-5-thiaprostaglandin $E_1$ methyl ester:-

A mixture of 59 mg of 2(R)-vinyl-3(S)-(3(S)-t-butyldimethylsilyloxy-5(S)-methyl-1-nonenyl)-4(R)-t-butyldimethylsilyloxycyclopentanone and 145 mg of 4-mercaptobutyric acid methyl ester was stirred in the presence of 15 mg of α,α'-azobis(isobutyronitrile) at 75°C for 5 hours. The resulting mixture was

purified by silica gel thin-layer chromatography to give 10.2 mg (yield 18%) of 17(S),20-dimethyl-5-thiaprostaglandin $E_1$ methyl ester 11,15-bis(t-butyldimethylsilyl)ether.

This compound was subjected to deprotecting reaction in the same way as in (iv) of Referencial Example 4.

The reaction mixture was purified by silica gel thin-layer chromatography to give 4.3 mg (yield 65%) of 17(S),20-dimethyl-5-thiaprostagrandin $E_1$ methyl ester.

TLC, Rf = 0.15 (cyclohexane : ethyl acetate = 1 : 4)

NMR ($\delta$ ppm in $CDCl_3$) :
0.90 (m, 6H), 1.0 - 18 (m, 13H),
2.0 - 3.1 (m, 12H), Mass (20eV, m/e) ;
396 ($M^+$ - 18)

Example 10

<u>Measurement of the action of inhibiting proliferation of L1212 leukemia cell:-</u>

L1210 leukemia cells were added to an RPMI medium containing 10% FCS (fetal calf serum), and the concentration of the cells was adjusted to $1 \times 10^5$ cells/ml. Each of the test compounds shown in Table 1 was dissolved in 99.5% ethanol. Prior to use, the final concentration of the ethanol solution was adjusted to less than 0.1%, and it was added to the culture medium. The culture medium was then maintained at 37°C in a stationary condition for 4 days. After the cultivation, the number of surviving cells was measured by dyeing with trypan flue. As a control, 0.1% ethanol was used. A dose-reaction curve was plotted from the ratios of proliferation against the control, and $IC_{50}$ was determined.

The results are shown in Table 1.

Table 1

| Test compound | $IC_{50}$ (µg/mℓ) |
|---|---|
| A mixture of (7E)-$\Delta^7$-5-thiaprostaglandin $E_1$ methyl ester of its 15-epimer | 0.4 |
| (7E)-$\Delta^7$-5-thiaprostaglandin $A_1$ methyl ester | 0.2 |
| 12-epimer of (7E)-$\Delta^7$-5-thiaprostaglandin $A_1$ methyl ester | 0.3 |
| 5-thiaprostaglandin $E_1$ methyl ester | 0.6 |

Example 11

<u>in vitro inhibitory activity of platelet aggregation:-</u>

The <u>in vitro</u> platelet aggregation imhibiting activity of the compounds of the invention were examined by using rabbits. Blood was withdrawn from the ear vein of Japanese domestic white male

rabbits weighing 2.5 to 3.5 kg. A mixture of a 3.8% trisodium citrate solution and the blood in a ratio of 1:9 was centrifuged at a speed of 1000 rpm for 10 minutes. The upper layer was separated as platelet-rich plasma (PRP). The lower layer was further centrifuged at a speed of 28000 rpm for 10 minutes. The upper layer was separated as platelet-poor plasma (PPP). The number of platelets was adjusted to 6 x $10^5$/μℓ to 7 x $10^5$/μℓ by diluting the PRP with PPP. 25 microliters of the test compounds prepared as shown below was added in an amount of 25 to 250 microliters of PRP after the adjustment, and the mixture was pre-incubated at 37°C for 2 minutes, and then 10 μM (final) of ADP was added. By using an aggregometer, changes in transmission were recorded.

The drug was dissolved in ethanol to a concentration of 10 mg/mℓ, and successively diluted with phosphate buffer (pH 7.4) prior to use.

The rate of inhibition of platelet aggregation was determined from the following equation.

$$\text{Inhibition rate (\%)} = \left(1 - \frac{T}{T_o}\right) \times 100$$

$T_o$: the transmittance of the system containing the phosphate buffer,

T : the transmittance of the system to which the test drug was added.

The minumum concentration of the drug which inhibited more than 50% of platelet aggregation was shown as an $IC_{50}$ value.

The results are shown in Table 2

Table 2

| Compound | $IC_{50}$ (μg/mℓ) |
|---|---|
| 17(S),20-dimethyl-5-thiaprostaglandin $E_1$ methyl ester | 0.33 |
| 17(R),20-dimethyl-5-thiaprostaglandin $E_1$ methyl ester | 0.36 |

Example 12

Measurement of inhibitry activity on ethanol ulcer:-

An ethanol ulcer test was carried out by the following processure.

SD-strain rats (body weight 200 to 220 g; 7 weeks old) were fasted for 24 hours, and a solution of each of the test compounds in physiological saline buffered with phosphate buffer at pH 7.5 was orally administered. Thirty minutes after the administration, 75% ethanol was orally administered in a dose of 1 mℓ/kg. One hour later, the abdomen was incised to isolate the stomach. The corpus was observed with a stereomicroscopy, and the lengths of ulcers were measured. The total of these lengths was defined as the ulcer index. The results are shown in Table 3. A control group was given physiological saline buffered with phosphate buffer (pH 7.5) and 75% ethanol.

The results are shown in Table 3.

Table 3

| Compounds | Dosage (p.o.; µg/kg) | Number of subject | Ulcer index (mm) | Ulcer inhibiting rate (%) | $ED_{50}$ (µg/kg) |
|---|---|---|---|---|---|
| Control | phosphate buffer solution (PBS) | 8 | 34.6 ±6.8 | - | - |
| 17(S),20-dimethyl-5-thiaprostaglandin $E_1$ methyl ester | 0.3 | 8 | 18.4 ±4.3 | 46.8 | 0.35 |
| | 1 | 8 | 0.8 ±0.6 | 97.7 | |
| | 3 | 8 | 0 | 100 | |
| Prostaglandin $E_1$ | PBS | 15 | 34.2 ±6.2 | - | 3.0 |
| | 3 | 9 | 17.1 ±5.5 | 50.0 | |
| | 10 | 6 | 1.3 ±0.8 | 96.2 | |

As shown in Table 3, the compounds of the present invention have stronger activity of anti-ulcer in comparison with inhibiting platelet aggregation activity in contrast Prostaglandin $E_1$ (inhibiting platelet aggregation activity of $PGE_1$ is 0.030 µg/mℓ ($IC_{50}$, invitro, ADP10µM)), and therefore have higher selectivity of anti-ulcer activity than $PGE_1$.

Example 13

<u>Measurement of the hypotensive activity:-</u>

The actions of the 5-thiaprostaglandin of the invention on the blood pressure was examined by

intravenous injection under anesthesia.

Male wister rats weighing about 250 g were used. Urethane (500 mg/kg) and α-chloralose (100 mg/kg) were intraperitoneally administered to the rats. The rats were anesthetized and fixed in place.

Each of the test compounds was dissolved in a small amount of ethanol and diluted with physiological saline to adjust the final ethanol concentration to not more than 5%. The solution was intravenously injected into the rats through a catheter inserted into the femoral vein.

The blood pressure of the rats was measured by a pressure transducer through a catheter inserted into the carotid artery of the rats.

The action of the test compound on the blood pressure was expressed as the dosage ($ED_{20}$, μ/kg) of the test compound which caused a 20% lowering of the mean blood pressure before administration of the compound.

The results are shown in Table 4.

Table 4

| Compound | $ED_{20}$ (μg/kg i.v.) |
|---|---|
| 17(S),20-dimethyl-5-thiaprostaglandin $E_1$ methyl ester | 2.7 |

Example 14

<u>Production of soft capsules:-</u>

One milligrams of 17(S),20-dimethyl-5-thiaprostalandin $E_1$ methyl ester obtained in Example 9 of

fractionated coconut oil and soft capsules were produced using a soft gelatin capsule making machine. Each of the capsules containing 1 μg of 17(S),20-dimethyl-5-thiaprostaglandin $E_1$ methyl ester.

Example 15

Preparation of an injectable solution:-

17(R),20-dimethyl-5-thiaprostaglandin $E_1$ methyl ester obtained in Example 7 as an active ingredient was dissolved in an amount of 60 μg in 5 mℓ of ethanol, and the solution was sterilized by passing it through a bacteria-holding filter. 0.1 mℓ of the solution was filled in each of 1-mℓ ampoules and the ampoules were sealed up. The contents of the ampoules are diluted, for example, with Tris-HCℓ buffer to 1 mℓ for injection.

Example 16

Production of tablets:-

Tablets were produced each of which contained the following ingredients.

| | |
|---|---|
| 17(S),20-dimethyl-5-thiaprostaglandin $E_1$ methyl ester | 10 μg |
| Lactonse | 250 mg |
| Potato starch | 70 mg |
| Polyvinyl pyrrolidone | 10 mg |
| Magnesium stearate | 5 mg |

The aforesaid 17(S),20-dimethyl-5-thiaprostalandin $E_1$ methyl ester obtained in Example 9, lactose and potato starch were mixed, and the mixture was wetted with a 20% ethanol solution of polyvinyl pyrrolidone. The wetted mixture was passed through a sieve. The resulting granules were mixed with magnesium stearate, and compression-molded into tablets.

WHAT WE CLAIM IS:

1. A 5-thiaprostaglandin of the following formula (1)

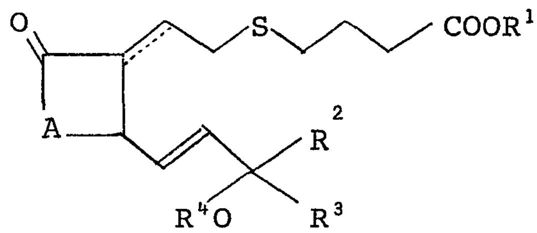

(1)

wherein A represents $-CH_2-\underset{OR^5}{\underset{|}{CH}}-$ or $-CH=CH-$ (cis) in which $R^5$ represents a hydrogen atom, a tri $(C_1-C_7)$hydrocarbon-silyl group, or a group forming an acetal linkage together with the oxygen atom of the hydroxyl group, the symbol ┄┄ represents a single or double bond, $R^1$ represents a hydrogen atom, a $C_1-C_{10}$ linear or branched alkyl group, or one equivalent of a cation, $R^2$ represents a hydrogen atom, or methyl group, $R^3$ represents an unsubstituted $C_3-C_8$ linear or branched alkyl group, a substituted $C_1-C_5$ linear or branched alkyl group substituted by a substituent selected from phenyl, phenoxy, $C_1-C_6$ alkoxy, $C_5-C_6$ cycloalkyl, which substituent may by substituted, or a substituted or unsubstituted alicyclic group, $R^4$ represents a hydrogen atom, a tri $(C_1-C_7)$ hydrocarbon-silyl group, or a group forming an acetal linkage together with the oxygen atom of the hydroxyl group; provided that when A is $-CH_2-\underset{OR^5}{\underset{|}{CH}}-$ and

$R^2$ is a hydrogen atom, $R^3$ is not an unsubstituted $C_3$-$C_8$ linear alkyl group.

2. The 5-thiaprostaglandin of claim 1 which is represented by the following formula (1)-a

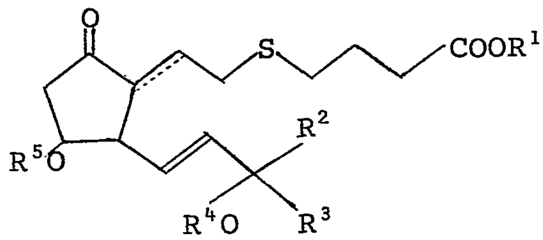

(1)-a

wherein $R^1$, $R^2$, $R^3$, $R^4$, $R^5$ and symbol ===== are as defined in claim 1.

3. The 5-thiaprostaglandin of claim 1 which is represented by the following formula (1)-b

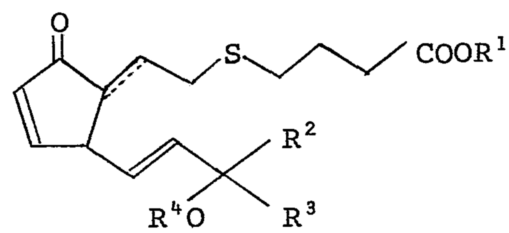

(1)-b

wherein $R^1$, $R^2$, $R^3$, $R^4$ and symbol ===== are as defined in claim 1.

4. The 5-thiaprostaglandin of any one of claims 1 to 3 wherein $R^3$ represents an n-pentyl, n-hexyl, 2-methyl-1-hexyl, 2-methyl-2-hexyl, cyclopentyl or cyclohexyl group.

5. The 5-thiaprostaglandin of any one of claims 1 to 3 wherein $R^4$ and $R^5$ represent a hydrogen atom, a tri($C_1$-$C_4$)alkylsilyl group, a 2-tetrahydropyranyl group, a 2-tetrahydrofuranyl group, a (2-methoxyethoxy)-methyl group, or a 6,6-dimethyl-3-oxa-2-oxo-bicyclo

[3.1.0]hex-4-yl group.

6. A process for producing the 5-thiaprostaglandin represented by the following formula (1)-a-1

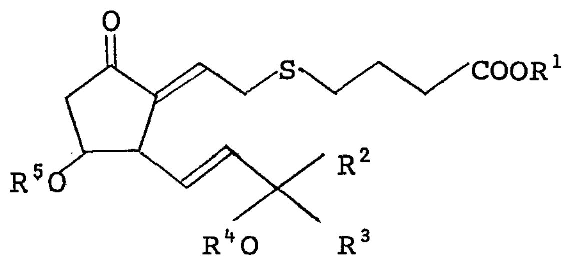

(1)-a-1

wherein $R^1$, $R^2$, $R^3$, $R^4$ and $R^5$ are defined as above,

which comprises reacting a 7-hydroxy-5-thiaprostaglandin represented by the following formula (11)

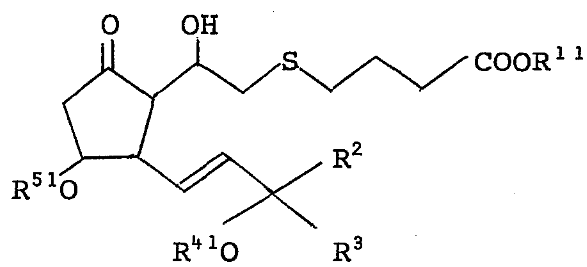

(11)

wherein $R^{11}$ represents a $C_1$-$C_{10}$ linear or branched alkyl group, $R^{41}$ and $R^{51}$ represents a tri($C_1$-$C_7$)hydrocarbonsilyl group or a group forming an acetal linkage together with the oxygen atom of the hydroxyl group, $R^2$ and $R^3$ are defined as above,

with a reactive derivative of an organic sulfonic acid in the presence of a basic compound to form the corresponding 7-organic sulfonyloxyprostaglandin, treating the 7-organicsulfonyloxyprostaglandin in the presence of a basic compound, and if required, subjecting the reaction product to a reaction of

removing the protective group, a reaction of hydrolysis, or a salt-forming reaction.

7. The process of claim 6 wherein the reactive derivative of the organic sulfonic acid is an organic sulfonic acid halide.

8. The process of claim 6 wherein the basic compound is an organic amine.

9. A process for producing the 5-thiaprostaglandin represented by the following formula

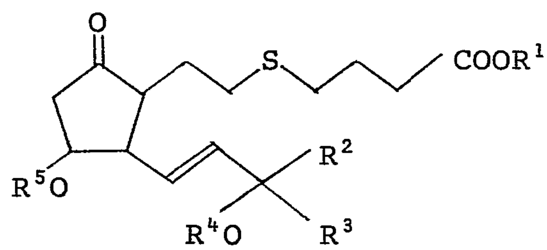

(1)-a-2

wherein $R^1$, $R^2$, $R^3$, $R^4$ and $R^5$ are as defined above,

which comprises selectively reducing the 5-thiaprostalandin represented by the following formula

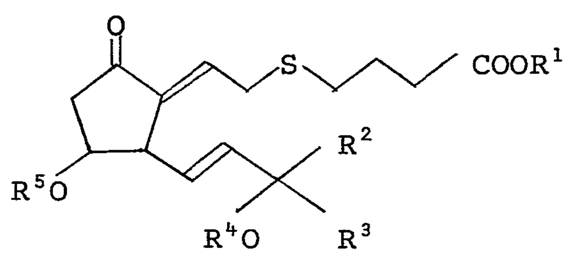

(1)-a-1

wherein $R^1$, $R^2$, $R^3$, $R^4$ and $R^5$ are defined as above,

and if required, subjecting the reaction product to a reaction of removing the protective group, a reaction of hydrolysis, or a salt-forming reaction.

10. The process of claim 9 wherein the selective

reduction is carried out by using metallic zinc or a reducing agent containing metallic zinc or by catalytic reduction in the presence of a Raney nickel catalyst, or using organic tin hydride.

11. A process for producing the 5-thiaprostalandin represented by the following formula (1)-a-2

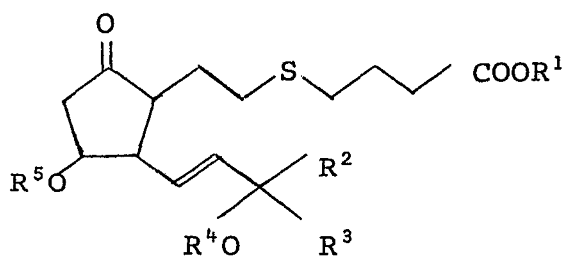

(1)-a-2

wherein $R^1$, $R^2$, $R^3$, $R^4$ and $R^5$ are defined as above,

which comprises reacting a α-vinylketone of the following formura (111)

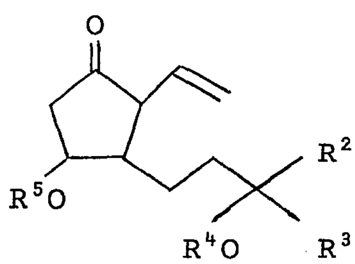

(111)

wherein $R^2$, $R^3$, $R^4$ and $R^5$ are defined as above,

and a thiol compound of the following formula

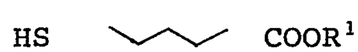

(1V)

wherein $R^1$ is defined as above,

in the presence of a radical generator, and if required, subjecting the reaction product to a reaction of removing the protective group, a reaction of hydrolysis or a salt-forming reaction.

12. A process for producing the 5-thiaprostaglandin represented by the following formula

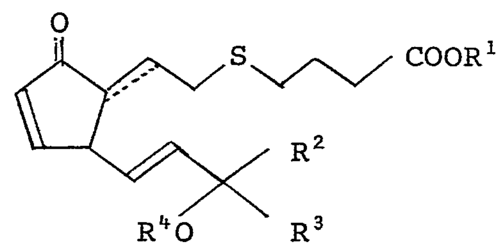

(1)-b

wherein $R^1$, $R^2$, $R^3$, $R^4$ and symbol ===== are defined as above,

which comprises subjecting a 5-thiaprostaglandin of the following formula (V)

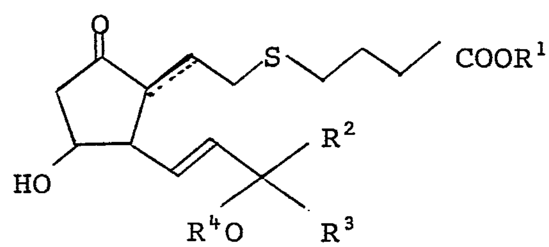

(V)

wherein $R^1$ and $R^4$ are defined as a above, $R^2$ represents a hydrogen atom or a methyl group, $R^3$ represents an unsubstituted $C_3$-$C_8$ linear or branched alkyl group, a substituted $C_1$-$C_5$ linear or branched alkyl group substituted by a substituent selected from phenyl, phenoxy, $C_1$-$C_6$ alkoxy, $C_5$-$C_6$ cycloalkyl, which substituent may be substituted, or a substituted or

unsubstituted alicyclic group,

to dehydration reaction, and if required, subjecting the reaction product to a reaction of removing the protective group, a reaction of hydrolysis, or a salt-forming reaction.

13. A pharmaceutical composition comprising a 5-thiaprostaglandin expressed by the following formula (1')

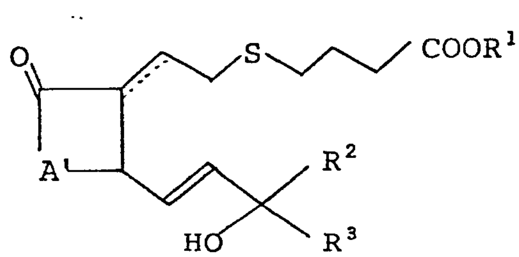

(1')

where $R^1$ and the symbol ┈┈┈ are defined as above, $A^1$ represents $-CH_2-\underset{\substack{| \\ OH}}{CH}-$ or $-CH=CH-$ (cis), $R^2$ represents a hydrogen atom or a methyl group, $R^3$ represents an unsubstituted $C_3-C_8$ linear or branched alkyl group, a substituted $C_1-C_5$ linear or branched alkyl group substituted by a substituent selected from phenyl, phenoxy, $C_1-C_6$ alkoxy, $C_5-C_6$ cycloalkyl, which substituent may be substituted, or a substituted or unsubstituted alicyclic group,

as an active ingredient and a pharmaceutically acceptable carrier.